Europäisches Patentamt

European Patent Office

Office européen des brevets

(11) Publication number: **0 488 800 B1**

## EUROPEAN PATENT SPECIFICATION

(45) Date of publication of patent specification: **11.10.95**

(51) Int. Cl.⁶: **C11C 3/10**, A23D 9/00, A23C 11/04

(21) Application number: **91311137.3**

(22) Date of filing: **29.11.91**

(54) **Corandomized fat compositions for infant formulas.**

(30) Priority: **30.11.90 US 620851**

(43) Date of publication of application:
**03.06.92 Bulletin 92/23**

(45) Publication of the grant of the patent:
**11.10.95 Bulletin 95/41**

(84) Designated Contracting States:
**AT BE CH DE DK ES FR GR IT LI LU NL SE**

(56) References cited:
**EP-A- 0 129 990**
**EP-A- 0 170 431**
**EP-A- 0 376 628**
**FR-A- 2 093 955**
**FR-A- 2 306 257**

**PATENT ABSTRACTS OF JAPAN vol. 011, no. 043 7 February 1987**

**JOURNAL OF THE AMERICAN OIL CHEMISTS' SOCIETY vol. 62, no. 2, 1985, CHAMPAIGN,ILL. pages 417 - 421; H. TRAITLER AND A. DIEFFENBACHER: 'PALM OIL AND PALMKERNEL OIL IN FOOD PRODUCTS'**

(73) Proprietor: **AMERICAN HOME PRODUCTS CORPORATION**
**Five Giralda Farms**
**Madison, New Jersey 07940-0874 (US)**

(72) Inventor: **Lien, Eric Louis**
**1 Anthony Drive**
**Malvern,**
**Pennsylvania 19355 (US)**
Inventor: **Tomarelli, Rudolph Michael**
**Box 146 R3**
**Phoenixville,**
**Pennsylvania 19460 (US)**

(74) Representative: **Connelly, Michael John et al**
**c/o Patent Department**
**Wyeth Laboratories**
**Huntercombe Lane South**
**Taplow**
**Maidenhead**
**Berkshire, SL6 0PH (GB)**

## Description

The invention disclosed herein comprises fat compositions primarily for use in nutritionally complete infant formulas in which the constituent palmitic acid oils and lauric acid oils are corandomized. The invention additionally includes such corandomized fat compositions with medium-chain triglycerides added, particularly for use in nutritional products for preterm or low birth weight infants. Such corandomization of two or more oils yields a mixture of triglycerides having a substantially different chemical makeup than that of the native oils themselves or than the native oils when randomized individually. Corandomization of the palmitic acid oils and the lauric acid oils affords an economical means of providing a very highly absorbed fat composition.

## Background Of The Invention

U.S. Patent No. 3,542,560, issued on November 24, 1970 to Tomarelli et al., discloses fat compositions for infant formulas having an increased portion of the palmitic acid in the beta (2) position of the triglyceride. This increase is obtained by blending lard, or a synthetic beta-monopalmitin, with the other oils comprising the fat composition, which have a relatively low portion of beta palmitic acid. Such other oils listed are corn, soy bean, palm, peanut, coconut, olive, babassu, cotton seed, oleo, and tallow. However, the use of lard is unacceptable in many areas of the world for religious reasons, and synthetic triglycerides are prohibitively expensive for large scale use. Thus, fat compositions for use in infant formulas are sought which are broadly acceptable on religious dietary grounds, are highly absorbed, have a fatty acid content similar to human milk and are economical to manufacture on very large scales.

Three more recent U.S. patents disclose all vegetable oil fat compositions for use in infant nutritional products with palm oil as the sole palmitic acid oil. These are U.S. Patent No. 4,282,265, issued on August 4, 1981, to Theuer and U.S. Patent Nos. 4,614,663 and 4,721,626, issued on September 30, 1986 and January 26, 1988, respectively, to Rule.

Most recently, European patent publication No. 0376628, published on July 4,1990, to American Home Products Corporation (Tomarelli) discloses all vegetable oil fat compositions in which the palmitic acid oil alone is randomized. This European patent publication also discloses all vegetable oil fat compositions including medium-chain triglycerides for use in infant nutritional products for preterm or low birthweight infants in which the palmitic acid oil alone is randomized.

The present invention differs from that of EP 0376628 in that at least one palmitic acid oil and one lauric acid oil are corandomized, which causes interesterification randomly between the fatty acids of the palmitic acid oil and the lauric acid oil. This corandomization of the two oils resulted in surprisingly superior absorbability to that found when the palm olein oil of the mixture was randomized. Additionally, in the fat compositions of the present invention oleo oil may be used as a palmitic acid oil to be corandomized with a lauric acid oil.

## Detailed Description of the Invention

This invention provides a corandomized fat composition particularly for use in a nutritionally complete infant formula, comprising

(a) 18-30%, calculated on the weight of the fat composition, of one or more lauric acid oils selected from coconut oil, babassu oil, and palm kernel oil;

(b) 20-40%, calculated on the weight of the fat composition, of one or more palmitic acid oils selected from oleo oil, palm oil, and palm olein oil;

(c) 13-34%, calculated on the weight of the fat composition, of one or more oleic acid oils selected from olive oil, safflower oleic oil, sunflower oleic oil, and canola oil; and

(d) 12-27%, calculated on the weight of the fat composition, of one or more linoleic acid oils selected from corn oil, cottonseed oil, safflower oil, soybean oil, and sunflower oil,

wherein at least the palmitic acid oils and the lauric acid oils are corandomized, the amounts of the oils being such that the fat composition contains, per 100 parts by weight of the total fatty acids present as triglycercides,

(i) 9-20 parts of lauric acid;

(ii) 10-25 parts of palmitic acid;

(iii) 2-10 parts of stearic acid;

(iv) 25-45 parts of oleic acid; and

(v) 11-28 parts of linoleic acid.

EP 0 488 800 B1

Preferred corandomized fat compositions of the invention are those wherein only the lauric acid oils and palmitic acid oils are corandomized. Also preferred are those fat compositions wherein only one oil of each type is used, and only one lauric acid oil and one palmitic acid oil are corandomized. The preferred palmitic acid oils are palm olein oil and oleo oil, of which palm olein oil is most preferred. The preferred lauric acid oil is coconut oil The preferred oleic acid oils are safflower oleic oil and sunflower oleic oil, and the preferred linoleic acid oil is soybean oil.

Preferred corandomized fat compositions of the invention comprise

(a) 20-29%, calculated on the weight of the fat composition, of a lauric acid oil selected from coconut oil, babassu oil, and palm kernel oil;

(b) 26-38%, calculated on the weight of the fat composition, of a palmitic acid oil selected from palm oil and palm olein oil;

(c) 14-30%, calculated on the weight of the fat composition, of an oleic acid oil selected from olive oil, safflower oleic oil, sunflower oleic oil, and canola oil; and

(d) 14-27%,calculated on the weight of the fat composition, of a linoleic acid oil selected from corn oil, cottonseed oil, safflower oil, soybean oil, and sunflower oil,

wherein the palmitic acid oil and the lauric acid oil are corandomized, the amounts of the oils being such that the fat composition contains, per 100 parts by weight of the total fatty acids present as triglycercides,

(i) 10-17 parts of lauric acid;

(ii) 11-22 parts of palmitic acid;

(iii) 3-8 parts of stearic acid;

(iv) 30-43 parts of oleic acid; and

(v) 13-23 parts of linoleic acid.

Especially preferred corandomized fat compositions of the invention are those wherein the oils comprise

(a) 22-28% coconut oil;

(b) 30-36% palm olein oil;

(c) 21-30% safflower oleic oil or sunflower oleic oil;and

(d) 14-22% soybean oil,

and wherein the fat composition contains, per 100 parts by weight of total fatty acid present as triglycerides,

(i) 8-14 parts of lauric acid;

(ii) 15-21 parts of palmitic acid;

(iii) 3-5 parts of stearic acid;

(iv) 33-43 parts of oleic acid, and

(v) 14-21 parts of linoleic acid.

Also especially preferred are corandomized fat compositions of the invention wherein the oils comprise

(a) 23-29% coconut oil;

(b) 30-37% oleo oil;

(c) 14-25% safflower oleic oil or sunflower oleic oil; and

(d) 18-25% soybean oil,

and particularly wherein the fat composition contains, per 100 parts by weight of total fatty acid present as triglycerides,

(i) 12-17 parts of lauric acid;

(ii) 15-21 parts of palmitic acid;

(iii) 3-5 parts of stearic acid;

(iv) 30-38 parts of oleic acid; and

(v) 16-22 parts of linoleic acid

Further particularly preferred corandomized fat compositions of the invention are those wherein the ratio of the palmitic acid oil to the lauric acid oil is between 65/35 palmitic acid oil/lauric acid oil and 40/60 palmitic acid oil/lauric acid oil. Especially preferred are fat compositions of the invention wherein the ratio of the palmitic acid oil to the lauric acid oil is between 60/40 palmitic acid oil/lauric acid oil and 45/55 palmitic acid oil/lauric acid oil.

This invention also provides a corandomized fat composition particularly for use in a nutritionally complete preterm (or low birthweight) infant formula, comprising

(a) 8-30%, calculated on the weight of the fat composition, of one or more lauric acid oils selected from coconut oil, babassu oil, and palm kernel oil;

(b) 8-32%,calculated on the weight of the fat composition, of one or more palmitic oils selected from oleo oil, palm oil, or palm olein oil;

3

EP 0 488 800 B1

(c) 8-30%, calculated on the weight of the fat composition, of one or more oleic acid oils selected from olive oil, safflower oleic oil, sunflower oleic oil, and canola oil;

(d) 10-30%, calculated on the weight of the fat composition, of one or more linoleic acid oils selected from corn oil, cottonseed oil, safflower oil, soybean oil, and sunflower oil; and

(e) 10-50%, calculated on the weight of the fat composition, of medium-chain triglycerides (MCT's), wherein at least the palmitic acid oils and the lauric acid oils are corandomized, the amounts of the oils being such that the fat composition contains, per 100 parts by weight of the total fatty acids present as triglycercides,

(i) 8-34 parts of caprylic acid;

(ii) 4-16 parts of capric acid;

(iii) 5-22 parts of palmitic acid;

(iv) 18-37 parts of oleic acid; and

(v) 7-19 parts of linoleic acid.

Preferred corandomized, preterm fat compositions of the invention are those wherein only the lauric acid oils and palmitic acid oils are corandomized. Also preferred are those preterm fat compositions wherein only one oil of each type is used, and only one lauric acid oil and one palmitic acid oil are corandomized. The preferred palmitic acid oils are palm olein oil and oleo oil, of which palm olein oil is most preferred. The preferred lauric acid oil is coconut oil The preferred oleic acid oils are safflower oleic oil and sunflower oleic oil, and the preferred linoleic acid oil is soybean oil

Preferred corandomized, preterm fat compositions of the invention comprise

(a) 15-29 %, calculated on the weight of the fat composition, of a lauric acid oil selected from coconut oil, babassu oil, and palm kernel oil;

(b) 15-32 %, calculated on the weight of the fat composition, of a palmitic oil selected from palm oil and palm olein oil;

(c) 8-30 %, calculated on the weight of the fat composition, of an oleic acid oil selected from olive oil, safflower oleic oil, sunflower oleic oil, and canola oil;

(d) 15-27%, calculated on the weight of the fat composition, of a linoleic acid oil selected from corn oil, cottonseed oil, safflower oil, soybean oil, and sunflower oil; and

(e) 10-30%, calculated on the weight of the fat composition, of medium-chain triglycerides (MCT's), wherein the palmitic acid oil and the lauric acid oil are corandomized, the amounts of the oils being such that the fat composition contains, per 100 parts by weight of the total fatty acids present as triglycercides,

(i) 8-25 parts of caprylic acid;

(ii) 4-12 parts of capric acid;

(iii) 7-20 parts of palmitic acid;

(iv) 25-38 parts of oleic acid; and

(v) 12-20 parts of linoleic acid.

Especially preferred preterm, corandomized fat compositions of the invention are those wherein the oils comprise

(a) 22-28% coconut oil;

(b) 20-30% palm olein oil;

(c) 19-30% safflower oleic oil or sunflower oleic oil; and

(d) 14-22% soybean oil; and

(e) 10-30% MCT's,

wherein the coconut oil and the palm olein oil are corandomized and wherein the fat composition contains, per 100 parts by weight of total fatty acid present as triglycerides,

(i) 8-20 parts of caprylic acid;

(ii) 4-8 parts of capric acid;

(iii) 10-17 parts of palmitic acid;

(iv) 26-36 parts of oleic acid; and

(v) 12-20 parts of linoleic acid.

Also especially preferred are corandomized, preterm fat compositions of the invention wherein the oils comprise

(a) 23-29% coconut oil;

(b) 20-30% oleo oil;

(c) 14-25% safflower oleic oil or sunflower oleic oil; and

(d) 18-25% soybean oil; and

(e) 10-30% MCT's,

wherein the coconut oil and the oleo oil are corandomized,

4

and particularly wherein the fat composition contains, per 100 parts by weight of total fatty acid present as triglycerides,

(i) 8-25 parts of caprylic acid;

(ii) 4-12parts of capric acid;

(iii) 7-20 parts of palmitic acid;

(iv) 30-38 parts of oleic acid; and

(v) 14-20 parts of linoleic acid.

Further particularly preferred corandomized fat compositions of the invention are those wherein the ratio of the palmitic acid oil to the lauric acid oil is between 65/35 palmitic acid oil/lauric acid oil and 40/60 palmitic acid oil/lauric acid oil. Especially preferred are fat compositions of the invention wherein the ratio of the palmitic acid oil to the lauric acid oil is between 60/40 palmitic acid oil/lauric acid oil and 45/55 palmitic acid oil/lauric acid oil.

The corandomized palmitic acid and lauric acid oils of the present invention are mixtures of triglycerides having unique chemical structures. In native fats and oils, the various fatty acids are positioned, i.e. esterified, on one of the three hydroxy groups of the glycerol molecule in an ordered pattern that is characteristic of the particular fat or oil. In general, the long chain saturated fatty acids, C16-C18, are predominantly on the 1 and 3 position, the mono and polyunsaturated fatty acids on the 2 or middle position of the triglyceride molecule. A second distributional characteristic of the fatty acids on the glycerol backbone that exists in nature results in a very large percentage of the triglycerides being so-called mixed triglycerides, i.e. each of the three fatty acids, or at least two, are different. There is only a small amount of simple triglycerides, those in which the three hydroxy groups are esterified with the same fatty acids, e.g. tripalmitin (C16), triolein (C18), etc.

Chemical interesterification, also called randomization (since it alters the non-random distribution of nature), is accomplished by heating the fat or oil for a short period of time, usually with a catalyst such as sodium methylate. The fatty acids leave their natural position on the triglyceride and rearrange in a random fashion, i.e., equally on each of the three positions. Thus, one-third of each individual fatty acid is on the one position, one-third on the two, and one-third on the three position of the triglycerides. Randomization of an individual native fatty acid oil also results in an increase in the content of simple triglycerides, or in the case of a palmitic acid oil, of triglycerides consisting only of the long chain saturated fatty acids palmitic and stearic acids. For example, when palm oil or palm olein oil is randomized alone, there is an increase in the amount of palmitic-stearic triglycerides from approximately 3% in the native oils to 11% in the individually randomized oils. Such long chain, completely saturated triglycerides are particularly poorly absorbed.

Corandomization of a lauric acid oil with a palmitic acid oil substantially alters the chemical makeup of the independent native oils because the fatty acids of both oils intermix with one another on the triglyceride. For example, listed below in Table I is the fatty acid makeup of coconut oil (a lauric acid oil) and of palm olein oil (a palmitic acid oil):

Table I

| Fatty Acid Composition of Coconut Oil and Palm Olein Oil | | |
|---|---|---|
| Fatty Acid | Percent in Coconut Oil | Percent in Palm Olein oil |
| Lauric (C12) | 53.8 | 0.3 |
| Myristic(C14) | 17.5 | 1.0 |
| Palmitic (C16) | 7.3 | 38.5 |
| Stearic (C18) | 2.0 | 4.5 |
| Oleic(C18:1) | 5.6 | 43.7 |
| Linoleic (C18:2 | 0.1 | 10.8 |

From this list it can be seen that palm olein oil has almost no lauric acid or myristic acid. Coconut oil, on the other hand, contains over 50% lauric acid and a fairly high percentage of myristic acid also. Thus, when coconut and palm olien oil are corandomized, there are many resulting triglyceride molecules which have a mixture of palmitic acid and either lauric acid or myristic acid or both - which cannot be the case when palm olein oil is randomized alone.

Thus, the resulting corandomized palm olein and coconut oil is not the same chemical entity as the mixture of nonrandomized palm olein and coconut oils or the mixture of randomized palm olein oil and nonrandomized coconut oil. Native palm olein oil is a collection of triglycerides of defined structures. Corandomized palm olein oil-coconut oil is a collection of triglycerides of entirely different chemical structures. The physical, biochemical and nutritional properties of the three oils/mixtures of oils are different.

The effect of corandomization on the positional distribution of fatty acids of native palm olein and coconut oils is presented in Table II. The alteration in the positional distribution of the fatty acids is indicated by the change in the proportion of the fatty acids in the 2-position. After randomization, theoretically one-third of each fatty acid should be in the 2-position. In practice, however, not all of the fatty acids are randomized equally or completely under the specific randomization conditions employed or at the determined endpoint of the run.

Table II

| Effect Of Corandomization On the Positional Distribution Of The Fatty Acids | | | | |
|---|---|---|---|---|
| Fattyacid** | Non-randomized 56% PO/44%CoCo* | | Corandomized 56% PO/44%CoCo | |
| | % FA | % in 2-pos. | % FA | % in 2-pos. |
| C8 | 5.6 | 4.8 | 5.0 | 32.0 |
| C10 | 3.9 | 21.4 | 3.6 | 37.0 |
| C12 | 25.6 | 66.3 | 25.0 | 34.1 |
| C14 | 9.5 | 23.9 | 9.4 | 42.6 |
| C16 | 24.2 | 10.1 | 24.7 | 34.0 |
| C18 | 3.2 | 12.5 | 3.3 | 37.4 |
| C18:1 | 22.2 | 35.1 | 23.1 | 28.1 |
| C18:2 | 5.8 | 40.8 | 5.9 | 28.8 |

* CoCo = coconut oil and PO = palm olein oil
** See table IV for the names of the fatty acids

Corandomized palm olein and coconut oil also differs importantly in its biochemical properties from a mixture of native palm olein and coconut oils. This difference is particularly significant for use in infant nutritional products. In the digestion of triglycerides in the intestine, pancreatic lipase hydrolyzes the fatty acids at the 1 and 3 position, resulting in two free fatty acids and a 2-monoglyceride containing the fatty acid of the glyceride 2 position. A long chain saturated fatty acid is less well absorbed as a free fatty acid than if it is present in the gut as a 2-monoglyceride.

Palmitic acid is the major saturated fatty acid of human milk triglycerides. It is a long chain, C16, fatty acid. Long chain fatty acids are not as well absorbed as short chain or unsaturated fatty acids, yet the palmitic acid of human milk is well absorbed because the palmitic acid of human milk is predominantly in the 2-position, and, after intestinal digestion, the majority of the palmitic acid is present in the intestine as the more readily absorbed 2-monopalmitin.

As seen in Table II above, corandomized palm olein oil/coconut oil has triple the amount of palmitic acid in the 2 position of the triglyceride as does a mixture of native palm olein oil and native coconut oil. Accordingly, the nutritional value of the corandomized fat compositions of the invention is significantly improved with respect to prior all vegetable oil fat compositions which use only the native palmitic acid oils.

Corandomization may be accomplished by heating from 0.5 to 4 hours, preferrably 0.5 to 2 hours, at temperatures from 100-140°C, preferably 110-130°C, with 0.05-0.50 percent, preferably 0.05-0.15 percent, of sodium methylate present. The end point of the corandomization process should provide palmitic acid at least 27%, and preferrably 33%, in the 2 position of the triglycerides.

The present invention also provides a nutritionally complete food product adapted for human infant nutrition containing the fat compositions according to the invention, as fully described above. Such food product comprises the fat composition, a protein source, a carbohydrate source, and appropriate levels of vitamins, minerals and other nutritional factors. The product may be a ready-to-feed liquid, or in the form of a powder or concentrated liquid adapted to provide a ready-to-feed form by the addition of water and stirring. The product preferably contains 2.2 to 4.0 g, advantageously about 3.6 g of a fat composition of the invention; 1.2 to 3.0 g, advantageously about 1.5 g of protein; and 6 to 9 g of carbohydrate - per 100 ml of the ready-to-feed liquid formula supplying preferably 60-75 kcal per 100 ml.

As protein sources there may be mentioned casein, salts of casein (e.g. potassium caseinate), whey protein concentrate, soybean protein isolate, cow's milk protein, or hydrolyzed whey, casein or soy protein.

Cow's milk protein differs from that of human milk in the proportions present as casein and whey protein. Cow's milk has about 80% casein and 20% whey proteins, whereas human milk has about 40% casein and about 60% whey proteins. Accordingly, the protein used may be adapted to simulate that of human milk by supplementing cow's milk protein with an appropriate amount of whey protein. Because whey contains a very high proportion of the minerals of milk, the whey is subjected to demineralisation, in particular by electrodialysis or ultrafiltration, to prepare whey protein. When a milk-free diet for infants who are intolerant of cow's milk protein is desired, the protein source may be isolated soy protein or hydrolyzed casein or whey protein. The proteins may be used in combination.

As a carbohydrate source lactose is generally preferred in formulas for normal, healthy infants. However, lactose would be contraindicated for infants suffering from galactosemia, lactose intolerance, or cow's milk protein intolerance. (In the latter case, the lactose may contain traces of cow's milk protein.) Where a milk-free diet is desired, the carbohydrate source may be sucrose, corn syrup solids (glucose polymers), or a combination of corn syrup solids with sucrose. The carbohydrates may also be used in combination.

Additionally, the food product (infant formula) would contain nutritionally acceptable quantities of the following minerals and vitamins: calcium, phosphorus, potassium, sodium, chloride, magnesium, iron, copper, zinc, manganese, iodine and selenium; and vitamin A, vitamin D, vitamin E, vitamin $K_1$, vitamin $B_1$, vitamin $B_2$, vitamin $B_6$, vitamin $B_{12}$, vitamin C, pantothenic acid, niacin, folic acid, biotin, choline and inositol. The food product could contain other nutritional factors, such as taurine, carnitine, nucleotides, and a source of long chain polyunsaturated fatty acids.

The present invention also provides a nutritionally complete food product adapted for the feeding of preterm or low birthweight infants, said product containing a fat composition according to the invention as fully described above. The product may be a ready-to-feed liquid or a powder or a concentrated liquid adapted to provide the ready-to-feed form by the addition of water and stirring. The product preferrably contains, per 100 ml of ready-to-feed formula, 1.5 - 2.5 g of protein, preferably 2.0 - 2.2 g of whey predominant protein; 2.2 -6.0 g of fat, preferably 3.5 - 4.4 g of the preferred corandomized fat blend of the present invention; and 4.7 - 11.0 g of carbohydrate, preferrably 7.0 - 8.6 g consisting of approximately equal parts of lactose and glucose polymers, said amounts supplying preferably 65 - 85 kcal / 100ml. Additionally, the preterm food product contains the vitamins, minerals and other nutritional factors described above for the term formula, but in amounts suitable for the preterm or low birthweight infant.

The invention includes a process for the preparation of the fat composition by blending the components (a), (b), (c), and (d) [and (e) for the preterm fat composition] together in such proportions that the resultant composition has the required composition of fatty acids. Additionally, an emulsifying agent such as lecithin or diglycerides, in an amount up to 2 percent of the total weight of the fat composition, may be blended into the fat mixture. Soy bean lecithin concentrate is commonly used, and since the concentrate contains essentially the same amount of fatty acids as in soybean oil, in the examples of fat blends presented below, 1 percent of soybean lecithin concentrate is included in the listed amounts of soybean oil. The proportions of the oils to be used can be calculated from the fatty acid profiles of the individual oil components. The blending is preferably performed at a blending temperature above the melting point of the fat mixture, whereby each component oil is in the liquid phase. The heating of the oils to the blending temperature and the mixing of the oils in a conventional mixing apparatus should be carried out with careful temperature control. A blending temperature within the range of about 36°C to 50°C may be used. Oil soluble vitamins are normally dissolved in the fat composition as a preliminary step.

To prepare the nutritionally complete food product the completed fat mixture is mixed with the other components which have been separately combined. The combination is then emulsified. Processing to a final ready-to-feed liquid, concentrated liquid or powder may be carried out in a conventional manner.

The practice of the invention is further represented by the following examples:

## Example 1

Table III presents eight fat blends of the invention, utilizing only the four preferred fat ingredients: Blends A and B represent preferred fat blends utilizing palm olein oil. Blends C and D represent preferred fat blends utilizing oleo oil. In Table III, the fatty acid totals do not add up to 100% because only the major fatty acids are included. The fatty acid percentage values used elsewhere in the description of the fat compositions of the invention are arrived at in a similar manner. The ratios of lauric acid oil to palmitic acid oil of fat blends A to H of Table III are shown in Table IIIa.

7

EP 0 488 800 B1

Table III

| Corandomized Fat Blends With Preferred Oils And Their Fatty Acid Compositions | | | | | | | | |
|---|---|---|---|---|---|---|---|---|
| Oils | Palm Olein | | Oleo | | Palm Olein | | Oleo | |
| | | | | | Low | Hi | Low | Hi |
| | A | B | C | D | E | F | G | H |
| Coconut | 25* | 27* | 27* | 25* | 28* | 20* | 28* | 26* |
| Palm olein | 32* | 35* | - | - | 26* | 38* | - | - |
| Oleo | - | - | 35* | 32* | - | - | 26* | 38* |
| Safflower oleic | 28 | 15 | 15 | 28 | 29 | 27 | 25 | 15 |
| Soybean | 15 | 23 | 23 | 15 | 17 | 15 | 21 | 21 |
| fatty acids** | | | | | | | | |
| C8 | 1.7 | 1.9 | 1.9 | 1.7 | 1.9 | 1.4 | 1.9 | 1.8 |
| C10 | 1.3 | 1.4 | 1.4 | 1.3 | 1.4 | 1.0 | 1.4 | 1.3 |
| C12 | 13.6 | 14.7 | 14.6 | 13.5 | 15.2 | 11.0 | 15.1 | 14.0 |
| C14 | 4.8 | 5.1 | 5.8 | 5.4 | 5.2 | 3.9 | 5.7 | 5.7 |
| C16 | 17.0 | 18.2 | 13.4 | 12.5 | 15.1 | 18.9 | 11.6 | 13.9 |
| C18 | 3.0 | 3.2 | 7.3 | 6.8 | 2.9 | 3.2 | 6.0 | 7.1 |
| C16:1 | 0.1 | 0.1 | 1.0 | 0.9 | 0.1 | 0.1 | 0.8 | 1.1 |
| C18:1 | 40.6 | 34.1 | 35.0 | 41.5 | 39.4 | 42.2 | 38.1 | 35.9 |
| C18:2 | 16.0 | 18.8 | 15.9 | 13.3 | 16.6 | 16.5 | 16.0 | 14.8 |
| C18:3 | 0.9 | 1.4 | 1.5 | 1.0 | 1.0 | 0.9 | 1.4 | 1.4 |

* Oils corandomized
** See Table IV for the names of the fatty acids

Table IIIa

| Ratio Of Palmitic Acid Oil To Lauric Acid Oil | | |
|---|---|---|
| Blend | Percent Total Palmitic and Lauric | Palmitic/Lauric |
| A | 57 | 56.0/44.0 |
| B | 62 | 56.4/43.6 |
| C | 62 | 56.4/43.6 |
| D | 57 | 56.1/43.9 |
| E | 54 | 48.0/52.0 |
| F | 58 | 65.5/34.5 |
| G | 54 | 48.2/51.8 |
| H | 64 | 59.0/41.0 |

Table IV shows the ranges of the fatty acid composition of human milk. These ranges were taken from 11 published reports from the U.S., Great Britain, Canada, West Germany, Australia and Finland from 1965-1983. Further variances from these ranges will be found in other geographic areas, for example, where the diet is largely vegetarian or where fish or other seafoods are a major food source. The fat compositions of the invention have a fatty acid pattern reasonably similar to that of human milk.

8

Table IV

| Human Milk Fatty Acid Ranges | | |
|---|---|---|
| Fatty Acid | | Ranges Reported |
| C8 | Caprylic | 0.1 |
| C10 | Capric | 0.8 - 1.6 |
| C12 | Lauric | 3.1 - 6.3 |
| C14 | Myristic | 5.1 - 7.4 |
| C16 | Palmitic | 20.2 - 25.2 |
| C18 | Stearic | 5.5 - 10.4 |
| C16:1 | Palmitoleic | 3.7 - 4.1 |
| C18:1 | Oleic | 29.4 - 46.9 |
| C18:2 | Linoleic | 7.2 - 15.6 |
| C18:3 | Linolenic | 0.7 - 2.0 |

## Example 2

Table V below gives seven further examples (I-O) of corandomized fat blends of the invention. These examples utilize the different oils in each group in the preferred amount for that particular group. Here, and in other tables herein, the fatty acids are listed by the number of carbon atoms in the chain, also noting positions of unsaturation, according to the conventional practice. Reference may be made to Table IV above, "Human Milk Fatty Acid Ranges", for correlation of the name of the fatty acid with conventional numerical designation.

Table V

| Preferred Corandomized Fat Blends With Other Oils Within The Class | | | | | | | | |
|---|---|---|---|---|---|---|---|---|
| Lauric acid oils | A** | I | J | K | L | M | N | O |
| coconut | 25* | 25* | | 25* | | | | |
| babassu | | | 25* | | 25* | 25* | | |
| palm kernel | | | | | | | 25* | 25* |
| Palmitic acid oils | | | | | | | | |
| palm olein | 32* | | 32* | | | | 32* | |
| palm | | | | 32* | 32* | | | |
| oleo | | 32* | | | | 32* | | 32* |
| Oleic acid oils | | | | | | | | |
| safflower oleic | 28 | | 28 | | | | 28 | |
| canola | | | | 28 | 28 | | | 28 |
| sunflower oleic | | 28 | | | | 28 | | |
| Linoleic acid oils | | | | | | | | |
| soy | 15 | | 15 | | | | 15 | |
| safflower | | | | 15 | 15 | | | |
| corn | | 15 | | | | 15 | | 15 |
| Fatty acids*** | | | | | | | | |
| C12 | 13.6 | 13.5 | 11.4 | 13.5 | 11.3 | 11.3 | 12.9 | 12.7 |
| C14 | 4.8 | 5.3 | 4.8 | 4.8 | 4.8 | 5.3 | 5.0 | 5.6 |
| C16 | 17.0 | 12.0 | 17.0 | 18.1 | 18.1 | 12.0 | 17.3 | 13.0 |
| C18 | 3.0 | 6.9 | 3.5 | 3.3 | 3.8 | 7.4 | 3.0 | 6.5 |
| C18:1 | 40.6 | 43.6 | 42.7 | 32.5 | 34.6 | 45.7 | 42.8 | 38.9 |
| C18:2 | 16.0 | 12.0 | 16.0 | 21.7 | 21.7 | 12.0 | 16.3 | 16.7 |
| C18:3 | 0.9 | 0.3 | 0.9 | 2.3 | 2.3 | 0.3 | 0.9 | 2.5 |

\* oils corandomized

\*\* also in Table III

\*\*\* See Table IV for the names of the fatty acids

## Example 3

Tables VI and VII below demonstrate the significant reductions in excretion of fat obtained with the corandomized fat blends of the invention. Table VI shows the reduction in excretion of the total fatty acids and of the palmitic acid itself from a diet containing corandomized coconut-palm olein oil compared to one containing the same ratio of nonrandomized coconut and palm olein oils. Table VII shows the reduction in excretion of palmitic acid from a diet containing corandomized coconut-palm olein oil compared to one containing the same ratio of a mixture of randomized palm olein oil and native coconut oil. The excretion data given below was obtained on young male rats according to the method described in U.S. Patent No. 3,542,560, issued on November 24, 1970, to Tomarelli et al., under "Part II" of the Example, at column 4, lines 34-73. Despite the fact that the rat absorbs fats very effeciently, marked differences in the fecal excretion of fat due to corandomization are readily demonstrated.

## Table VI
### Fat Excretion Of Mixtures Of Native Coconut And Palm Olein Oils Versus That Of Corandomized Coconut-Palm Olein Oil

| Ratio CoCo/PO | Native Oils | Corand. Oil | Native Oils | Corand. Oil |
|---|---|---|---|---|
| | ---------------------- Percent Excretion ---------------------------- | | | |
| | ----Total Fatty Acids---- | | ----- Palmitic Acid ------- | |
| 53/47 | 3.5 ±1.3 | 1.3 ±0.2 | 9.7 ±0.7 | 3.2 ±0.6 |
| 44/56 | 5.0 ±0.3 | 2.1 ±0.3 | 13.2 ±0.7 | 5.3 ±0.7 |
| 35/65 | 7.4 ±0.6 | 2.1 ±0.3 | 16.6 ±1.3 | 4.5 ±0.6 |
| 25/75 | 7.4 ±0.7 | 4.2 ±0.3 | 16.1 ±1.5 | 8.6 ±0.7 |

all differences are statistically significant

## Table VII
### Excretion Of Palmitic Acid From Corandomized Coconut-Palm Olein Oil Versus That For Coconut Oil Plus Randomized Palm Olein Oil

| Ratio Coco/PO | Coco + Rand. PO | Corand. Coco-PO |
|---|---|---|
| | ---------- Percent Excretion -------------- | |
| 53/47 | 11.5 ±1.4 | 3.2 ±0.6 |
| 44/56 | 10.0 ± 0.8 | 5.3 ±0.7 |
| 35/65 | 7.8 ±1.2 | 4.5 ±0.6 |
| 25/75 | 10.3 ±0.7 | 8.6 ±0.7 |

all differences are statistically significant

The data in Table VI demonstrates the surprising reductions in excretion of both total fatty acids and of palmitic acid found with the corandomized lauric acid-palmitic acid oil of the invention when compared to the same mixture of the native (nonrandomized) oils. These significant reductions in excretion are only partly explained by the increased amount of palmitic acid in the 2-position in the corandomized oil. These data also show the particularly signifcant reductions in excretion of both total fatty acid and of palmitic acid when the ratio of coconut oil/palm olein oil corandomized is 35/65 to 53/47. Again, these improvements due to the invention can only be partly explained by the increased amount of the palmitic acid in the 2-position of the corandomized oil.

The data in Table VII demonstrates a further surprising result with respect to the corandomized oils of the invention. This data demonstrates significant decreases in excretion of palmitic acid of a corandomized lauric acid-palmitic acid oil of the invention when compared to a similar mixture of native lauric acid oil and randomized palmitic acid oil. These results are indeed unexpected since the amount of palmitic acid in the 2-position is approximately 33% in both the corandomized oil and the mixed nonrandomized-randomized oils.

A possible explanation for these unexpected results lies in the change in the profile of the long chain saturated triglycerides of the corandomized oil compared to that of the mixture of one native and one randomized oil (ie. the palmitic acid oil). The amount of palmitic-stearic triglycerides in the corandomized mixtures may be calculated and is shown in Table VIII below.

Table VIII

| Percentages Of Long Chain Saturated Triglycerides Relative To The Proportions Of Coconut Oil And Palm Olein Oil That Are Corandomized | |
|---|---|
| Coconut/palm olein ratio | % triglycerides containing only palmitic and stearic acids |
| 53/47 | 1.4 |
| 44/36 | 2.2 |
| 35/65 | 3.2 |
| 25/75 | 4.4 |
| 0/100 | 11.0 |

From these calculations it can be seen that, as the proportion of coconut oil is descreased in the corandomized mixtures, the percentage of palmitic-stearic acid triglycerides increases from 1.4% to the 11% that results when the palm olein oil is randomized separately. Thus, the expecially favorable absorption (ie. reduced excretion) achieved with corandomization may be explained not only in the proportion of palmitic acid in the 2-position, but also, and apparently much more significantly, because of the reduced amount of the long chain saturated triglycerides of palmitic and stearic acids, which are poorly digested and absorbed.

## Example 4

Table IX below shows seven corandomized fat blends of the invention particularly for use in formulas for preterm or low birthweight infants. These fat blends use the preferred lauric and palmitic acid oils for corandomization.

Table IX

| Fat Blends For Preterm Infants | | | | | | | |
|---|---|---|---|---|---|---|---|
| Fat ingredient | S | T | U | V | W | X** | Y |
| Coconut oil | 30 | 21 | 12 | 28 | 25 | 27 | 20 |
| Palm Olein oil | 10 | 30 | 10 | 24 | 20 | 20 | 15 |
| Safflower Oleic oil | 25 | 21 | 15 | 8 | 20 | 25 | 20 |
| Soybean oil | 25 | 18 | 13 | 10 | 15 | 18 | 15 |
| MCT | 10 | 10 | 50 | 50 | 20 | 10 | 30 |
| Fatty acids*** | | | | | | | |
| C8 | 8.7 | 8.1 | 33.7 | 33.5 | 14.9 | 8.5 | 21.1 |
| C10 | 4.6 | 4.2 | 3.8 | 6.9 | 7.4 | 3.0 | 6.5 |
| C12 | 16.6 | 12.1 | 7.4 | 5.5 | 14.2 | 15.1 | 11.6 |
| C14 | 5.5 | 4.2 | 2.3 | 1.8 | 4.8 | 5.1 | 3.8 |
| C16 | 9.6 | 15.8 | 6.7 | 11.1 | 11.9 | 12.6 | 9.6 |
| C18 | 2.4 | 2.8 | 1.4 | 1.7 | 2.3 | 2.5 | 2.0 |
| C18:1 | 31.3 | 34.3 | 19.6 | 19.1 | 23.8 | 33.5 | 26.5 |
| C18:2 | 18.7 | 16.1 | 10.5 | 9.1 | 13.3 | 15.7 | 12.8 |
| C18:3 | 1.5 | 1.1 | 0.8 | 0.6 | 0.9 | 1.1 | 0.9 |

* MCT - medium chain triglycerides
** Preferred
*** See Table IV for the names of the fatty acids

Table X shows fat blends for preterm infants in which oleo oil is the palmitic acid oil corandomized with coconut oil.

Table X

| Fat Blends For Preterm Infants Containing Corandomized Oleo And Coconut Oil | | | | | | | |
|---|---|---|---|---|---|---|---|
| Fat Ingredient | AA | AB | AC | AD | AE | AF | AG |
| Coconut oil | 27 | 30 | 20 | 12 | 21 | 8 | 25 |
| Oleo oil | 20 | 10 | 15 | 10 | 30 | 24 | 20 |
| Safflower oleic oil | 25 | 25 | 20 | 15 | 21 | 8 | 20 |
| Soybean oil | 18 | 25 | 15 | 13 | 18 | 10 | 15 |
| MCT | 10 | 10 | 30 | 50 | 10 | 50 | 20 |
| Fatty acids * | | | | | | | |
| C8 | 8.5 | 8.7 | 21.1 | 33.7 | 8.0 | 33.4 | 14.9 |
| C10 | 4.4 | 4.6 | 10.2 | 16.0 | 4.1 | 15.8 | 7.4 |
| C12 | 14.8 | 16.4 | 11.3 | 7.3 | 11.5 | 5.1 | 13.8 |
| C14 | 5.4 | 5.6 | 4.0 | 2.4 | 4.6 | 2.1 | 5.0 |
| C16 | 9.8 | 8.2 | 7.5 | 5.3 | 11.6 | 7.8 | 9.1 |
| C18 | 4.9 | 3.6 | 3.7 | 2.6 | 6.3 | 4.6 | 4.7 |
| C16:1 | 0.6 | 0.3 | 0.5 | 0.3 | 0.9 | 0.7 | 0.6 |
| C18:1 | 34.5 | 31.7 | 27.2 | 20.1 | 35.7 | 20.2 | 29.8 |
| C18:2 | 14.2 | 17.9 | 11.7 | 9.7 | 13.9 | 7.3 | 11.8 |
| C18:3 | 1.2 | 1.5 | 1.0 | 0.8 | 1.2 | 0.7 | 1.0 |

\* See Table IV for the names of the fatty acids

## Example 5

Given below are two examples of the composition of a complete infant nutritional food product using a corandomized fat composition of the invention. In the examples, the preferred fat composition is used, but any corandomized palmitic acid oil-lauric acid oil fat blend of the invention may be used ("PO" below stands for palm olein oil, and "S-Oleic" stands for safflower oleic oil.)

Example 5 - Term Formulas

| | 1A | 1B |
|---|---|---|
| Protein | non-fat milk and demineralized whey | soy protein isolate \ |
| Fat (oils) | PO - 25%<br>Coconut - 32%<br>S.-Oleic - 28%<br>Soybean - 15% | PO - 25%<br>Coconut - 32%<br>S.-Oleic - 28%<br>Soybean - 15% |
| Carbohydrate | lactose | sucrose |

| Constituents | per liter | per liter |
|---|---|---|
| Energy kcal | 676 | 676 |
| Protein g | 15 | 21 |
| Fat g | 36 | 36 |
| Carbohydrate g | 72 | 69 |
| Water g | 904 | 898 |
| Linoleic Acid mg | 3300 | 3300 |
| Vitamin A IU | 2000 | 2000 |
| Vitamin D IU | 400 | 400 |
| Vitamin E IU | 9.5 | 9.5 |
| Vitamin K mcg | 55 | 100 |
| Thiamin (Vit B1) mcg | 670 | 670 |
| Riboflavin (Vit B2) mcg | 1000 | 1000 |
| Vitamin B6 mcg | 420 | 420 |
| Vitamin B12 mcg | 1.3 | 2 |
| Niacin mcg | 5000 | 5000 |
| Folic Acid (Folacin) mcg | 50 | 50 |
| Pantothenic Acid mcg | 2100 | 2100 |
| Biotin mcg | 15 | 35 |
| Vit C (Ascorbic Acid) mg | 55 | 55 |
| Choline mg | 100 | 85 |
| Inositol mg | 32 | 27 |
| Taurine mg | 40 | 40 |
| Carnitine mg | 37 | 8.5 |
| Nucleotide monophosphates mg | 29.5 | -- |
| Calcium mg | 420 | 600 |
| Phosphorus mg | 280 | 280 |
| Magnesium mg | 45 | 67 |
| Iron mg (w/wo) | 12.0/1.5 | 11.5 |
| Zinc mg | 5 | 5 |
| Manganese mcg | 150 | 150 |
| Copper mg | 470 | 470 |
| Iodine mcg | 60 | 60 |
| Sodium mg | 150 | 200 |
| Potassium mg | 560 | 700 |
| Chloride mg | 375 | 375 |

## Example 6

Given below are two examples of the composition of a complete preterm infant nutritional food product using a corandomized preterm fat composition according to the invention. In the examples, the preferred preterm fat composition is used, but any corandomized palmitic acid oil / lauric acid oil fat composition of the invention may be used. ("PO" below stands for palm olein oil, and "MCT" stands for medium-chain triglycerides.)

|  | 2A | 2B |
|---|---|---|
| Protein | non-fat milk and demineralized whey | Non-fat milk and demineralized whey |
| Fat (oils) | MCT - 10%<br>PO - 20%<br>Coconut - 27%<br>S.-Oleic - 25%<br>Soybean - 18% | MCT - 10%<br>Oleo - 25%<br>Coconut - 27%<br>S.-Oleic - 15%<br>Soybean - 23% |
| Carbohydrate | lactose and glucose polymers | lactose and glucose polymers |

| Constituents | per liter | per liter |
|---|---|---|
| Energy kcal | 810 | 810 |
| Protein g | 20 | 22.0 |
| Fat g | 44 | 42.1 |
| Carbohydrate g | 86 | 86.5 |
| Water g | 880 | 882 |
| Linoleic Acid mg | 4000 | 4050 |
| Vitamin A IU | 2400 | 8100 |
| Vitamin D IU | 480 | 2430 |
| Vitamin E IU | 15 | 36.5 |
| Vitamin K mcg | 70 | 105 |
| Thiamin (Vit B1) mcg | 800 | 2025 |
| Riboflavin (Vit B2) mcg | 1300 | 2835 |
| Vitamin B6 mcg | 500 | 2025 |
| Vitamin B12 mcg | 2 | 3.2 |
| Niacin mcg | 6300 | 36450 |
| Folic Acid (Folacin) mcg | 100 | 284 |
| Pantothenic Acid mcg | 3600 | 12150 |
| Biotin mcg | 18 | 16.2 |
| Vit C (Ascorbic Acid) mg | 70 | 284 |
| Choline mg | 127 | 64.8 |
| Inositol mg | 32 | 200 |
| Taurine mg | 48 | 48 |
| Carnitine mg | 49 | 59 |

Example 6 - Preterm Formulas (continued)

| Constituents | per liter | per liter |
|---|---|---|
| Nucleotide monophosphates mg | 29.5 | 29.5 |
| Calcium mg | 750 | 1000 |
| Phosphorus mg | 400 | 600 |
| Magnesium mg | 70 | 81 |
| Iron mg | 3 | 2.4 |
| Zinc mg | 8 | 10.5 |
| Manganese mcg | 200 | 105 |
| Copper mcg | 700 | 1417.5 |
| Iodine mcg | 83 | 81 |
| Sodium mg | 320 | 405 |
| Potassium mg | 750 | 972 |
| Chloride mg | 530 | 729 |

**Claims**

1. A corandomized fat composition particularly for use in a nutritionally complete infant formula, comprising

    (a) 18-30%, calculated on the weight of the fat composition, of one or more lauric acid oils selected from coconut oil, babassu oil, and palm kernel oil;
    (b) 20-40%, calculated on the weight of the fat composition, of one or two palmitic acid oils selected from oleo oil, palm oil, and palm olein oil;
    (c) 13-34%, calculated on the weight of the fat composition, of one or more oleic acid oils selected from olive oil, safflower oleic oil, sunflower oleic oil, and canola oil; and
    (d) 12-27%, calculated on the weight of the fat composition, of one or more linoleic acid oils selected from corn oil, cottonseed oil, safflower oil, soybean oil, and sunflower oil,
    wherein at least the palmitic acid oils and the lauric acid oils are corandomized, the amounts of the oils being such that the fat composition contains, per 100 parts by weight of the total fatty acids present as triglycercides,
    (i) 9-20 parts of lauric acid;
    (ii) 10-25 parts of palmitic acid;
    (iii) 2-10 parts of stearic acid;
    (iv) 25-45 parts of oleic acid; and
    (v) 11-28 parts of linoleic acid.

2. A corandomized fat composition according to claim 1 wherein only the palmitic acid oils and the lauric acid oils are corandomized.

3. A corandomized fat composition according to claim 2 wherein only one palmitic acid oil and one lauric acid oil are corandomized.

4. A corandomized fat composition according to claim 3 wherein the palmitic acid oil is oleo oil or palm olein oil and the lauric acid oil is coconut oil.

5. A corandomized fat composition according to claim 1 wherein only one of each type of oil is used.

6. A corandomized fat composition according to claim 1 wherein one palmitic acid oil is used, which is palm olein oil.

7. A corandomized fat composition according to claim 1 wherein one oleic acid oil is used, which is either sunflower oleic oil or safflower oleic oil.

8. A corandomized fat composition according to claim 1 wherein only one linoleic acid oil is used, which is soybean oil.

9. A corandomized fat composition particularly for use in a nutritionally complete infant formula, comprising

    (a) 20-29%, calculated on the weight of the fat composition, of a lauric acid oil selected from coconut oil, babassu oil, and palm kernel oil;
    (b) 26-38%, calculated on the weight of the fat composition, of a palmitic acid oil selected from palm oil and palm olein oil;
    (c) 14-30%, calculated on the weight of the fat composition, of an oleic acid oil selected from olive oil, safflower oleic oil, sunflower oleic oil, and canola oil; and
    (d) 14-27%, calculated on the weight of the fat composition, of a linoleic acid oil selected from corn oil, cottonseed oil, safflower oil, soybean oil, and sunflower oil,
    wherein at least the palmitic acid oil and the lauric acid oil are corandomized, the amounts of the oils being such that the fat composition contains, per 100 parts by weight of the total fatty acids present as triglycercides,
    (i) 10-17 parts of lauric acid;
    (ii) 11-22 parts of palmitic acid;
    (iii) 3-8 parts of stearic acid;
    (iv) 30-43 parts of oleic acid; and

16

(v) 13-23 parts of linoleic acid.

10. A corandomized fat composition particularly for use in a nutritionally complete preterm (or low birthweight) infant formula, comprising

(a) 8-30%, calculated on the weight of the fat composition, of one or more lauric acid oils selected from coconut oil, babassu oil, and palm kernel oil;

(b) 8-32%, calculated on the weight of the fat composition, of one or more palmitic acid oils selected from oleo oil, palm oil and palm olein oil;

(c) 8-30%, calculated on the weight of the fat composition, of one or more oleic acid oils selected from olive oil, safflower oleic oil, sunflower oleic oil, and canola oil;

(d) 10-30%,calculated on the weight of the fat composition, of one or more linoleic acid oils selected from corn oil, cottonseed oil, safflower oil, soybean oil, and sunflower oil; and

(e) 10-50%, calculated on the weight of the fat composition, of medium-chain triglycerides (MCTs).

wherein at least the palmitic acid oil and the lauric acid oil are corandomized, the amounts of the oils being such that the fat composition contains, per 100 parts by weight of the total fatty acids present as triglycercides,

(i) 8-34 parts of caprylic acid;

(ii) 4-16 parts of capric acid;

(iii) 5-22 parts of palmitic acid;

(iv) 18-37 parts of oleic acid; and

(v) 7-19 parts of linoleic acid.

11. A preterm, corandomized fat composition according to claim 10 wherein only the palmitic acid oils and the lauric acid oils are corandomized.

12. A preterm, corandomized fat composition according to claim 10 wherein only one palmitic acid oil and one lauric acid oil are corandomized.

13. A preterm, corandomized fat composition according to claim 12 wherein the palmitic acid oil is oleo oil or palm olein oil and the lauric acid oil is coconut oil.

14. A preterm, corandomized fat composition according to claim 10 wherein the palmitic acid oil is palm olein oil.

15. A preterm, corandomized fat composition according to claim 10 wherein only one of each type of oil is used.

16. A preterm, corandomized fat composition according to claim 10 wherein one oleic acid oil is used, which is either sunflower oleic oil or safflower oleic oil.

17. A preterm, corandomized fat composition according to claim 10 wherein only one linoleic acid oil is used, which is soybean oil.

18. A corandomized fat composition particularly for use in a nutritionally complete preterm (or low birthweight) infant formula, comprising

(a) 15-29%, calculated on the weight of the fat composition, of a lauric acid oil selected from coconut oil, babassu oil, and palm kernel oil;

(b) 15-32%, calculated on the weight of the fat composition, of a palmitic acid oil selected from palm oil and palm olein oil;

(c) 8-30%, calculated on the weight of the fat composition, of an oleic acid oil selected from olive oil, safflower oleic oil, sunflower oleic oil, and canola oil;

(d) 15-27%, calculated on the weight of the fat composition, of a linoleic acid oil selected from corn oil, cottonseed oil, safflower oil, soybean oil, and sunflower oil; and

(e) 10-30%, calculated on the weight of the fat composition, of medium-chain triglycerides (MCTs).

wherein at least the palmitic acid oil and the lauric acid oil are corandomized, the amounts of the oils being such that the fat composition contains, per 100 parts by weight of the total fatty acids present as triglycercides,

(i) 8-25 parts of caprylic acid;

(ii) 4-12 parts of capric acid;

(iii) 7-20 parts of palmitic acid;

(iv) 25-38 parts of oleic acid; and

(v) 12-20 parts of linoleic acid.

19. A corandomized fat composition according to claim 1 wherein the oils comprise

(a) 22-28% coconut oil;

(b) 30-36% palm olein oil;

(c) 21-30% safflower oleic oil or sunflower oleic oil; and

(d) 14-22% soybean oil,

and wherein the fat composition contains, per 100 parts by weight of total fatty acid present as triglycerides,

(i) 8-14 parts of lauric acid;

(ii) 15-21 parts of palmitic acid;

(iii) 3-5 parts of stearic acid;

(iv) 33-43 parts of oleic acid; and

(v) 14-21 parts of linoleic acid.

20. A preterm, corandomized fat composition according to claim 10 wherein the oils comprise

(a) 22-28% coconut oil;

(b) 20-30% palm olein oil;

(c) 19-30% safflower oleic oil or sunflower oleic oil; and

(d) 14-22% soybean oil; and

(e) 10-30% MCT's,

wherein the coconut oil and the palm olein oil are corandomized, and wherein the fat composition contains, per 100 parts by weight of total fatty acid present as triglycerides,

(i) 8-20 parts of caprylic acid;

(ii) 4-8 parts of capric acid;

(iii) 10-17 parts of palmitic acid;

(iv) 26-36 parts of oleic acid; and

(v) 12-20 parts of linoleic acid.

21. A nutrutionally complete food composition adapted for the nutrition of human infants, which comprises a fat composition as claimed in any one of claims 1 to 9 and 19, a source of protein, a source of carbohydrate, vitamins and minerals.

22. A nutritionally complete food composition adapted for the nutrition of preterm infants or low birth weight infants, which comprises a fat composition as claimed in any one of claims 10 to 18 and 20, a source of protein, a source of carbohydrate, vitamins and minerals.

**Patentansprüche**

1. Co-randomisierte Fettzusammensetzung, insbesondere zur Verwendung in einer Vollnahrungsmittelformel für Säuglinge, welche umfaßt:

(a) 18 bis 30 %, bezogen auf die Masse der Fettzusammensetzung, eines oder mehrerer Laurinsäureöle, ausgewählt aus Kokosnußöl, Babassuöl und Palmkernöl;

(b) 20 bis 40 %, bezogen auf die Masse der Fettzusammensetzung, eines oder zweier Palmitinsäureöle, ausgewählt aus Oleoöl, Palmöl und Palmoleinöl;

(c) 13 bis 34 %, bezogen auf die Masse der Fettzusammensetzung, eines oder mehrerer Oleinsäureöle, ausgewählt aus Olivenöl, Safloroleinöl, Sonnenblumenoleinöl und Canolaöl; und

(d) 12 bis 27 %, bezogen auf die Masse der Fettzusammensetzung, eines oder mehrerer Linolsäureöle, ausgewählt aus Maisöl, Baumwollsamenöl, Safloröl, Sojabohnenöl und Sonnenblumenöl,

worin zumindest die Palmitinsäureöle und die Laurinsäureöle co-randomisiert sind,

wobei die Mengen der Öle derart sind, daß die FettzusammenSetzung, pro 100 Masseteile der gesamten, als Triglyceride vorliegenden Fettsäuren, enthält:

(i) 9 bis 20 Teile Laurinsäure;

(ii) 10 bis 25 Teile Palmitinsäure;

(iii) 2 bis 10 Teile Stearinsäure;

18

(iv) 25 bis 45 Teile Oleinsäure; und

(v) 11 bis 28 Teile Linolsäure.

2. Co-randomisierte Fettzusammensetzung nach Anspruch 1, worin nur die Palmitinsäureöle und die Laurinsäureöle co-randomisiert sind.

3. Co-randomisierte Fettzusammensetzung nach Anspruch 2, worin nur ein Palmitinsäureöl und ein Laurinsäureöl co-randomisiert sind.

4. Co-randomisierte Fettzusammensetzung nach Anspruch 3, worin das Palmitinsäureöl Oleoöl oder Palmoleinöl ist, und das Laurinsäureöl Kokosnußöl ist.

5. Co-randomisierte Fettzusammensetzung nach Anspruch 1, worin nur einer von jedem Öl-Typ verwendet wird.

6. Co-randomisierte Fettzusammensetzung nach Anspruch 1, worin ein Palmitinsäureöl, das Palmoleinöl ist, verwendet wird.

7. Co-randomisierte Fettzusammensetzung nach Anspruch 1, worin ein Oleinsäureöl, das entweder Sonnenblumenoleinöl oder Safloroleinöl ist, verwendet wird.

8. Co-randomisierte Fettzusammensetzung nach Anspruch 1, worin nur ein Linolsäureöl, das Sojabohnenöl ist, verwendet wird.

9. Co-randomisierte Fettzusammensetzung, insbesondere zur Verwendung in einer Vollnahrungsmittelformel für Säuglinge, welche umfaßt:
(a) 20 bis 29 %, bezogen auf die Masse der Fettzusammensetzung, eines Laurinsäureöls, ausgewählt aus Kokosnußöl, Babassuöl und Palmkernöl;
(b) 26 bis 38 %, bezogen auf die Masse der Fettzusammensetzung, eines Palmitinsäureöls, ausgewählt aus Palmöl und Palmoleinöl;
(c) 14 bis 30 %, bezogen auf die Masse der Fettzusammensetzung, eines Oleinsäureöls, ausgewählt aus Olivenöl, Safloroleinöl, Sonnenblumenoleinöl und Canolaöl; und
(d) 14 bis 27 %, bezogen auf die Masse der Fettzusammensetzung, eines Linolsäureöls, ausgewählt aus Maisöl, Baumwollsamenöl, Safloröl, Sojabohnenöl und Sonnenblumenöl,
worin zumindest das Palmitinsäureöl und das Laurinsäureöl co-randomisiert sind,
wobei die Mengen der Öle derart sind, daß die FettzusammenSetzung, pro 100 Masseteile der gesamten, als Triglyceride vorliegenden Fettsäuren, enthält:
(i) 10 bis 17 Teile Laurinsäure;
(ii) 11 bis 22 Teile Palmitinsäure;
(iii) 3 bis 8 Teile Stearinsäure;
(iv) 30 bis 43 Teile Oleinsäure; und
(v) 13 bis 23 Teile Linolsäure.

10. Co-randomisierte Fettzusammensetzung, insbesondere zur Verwendung in einer Vollnahrungsmittelformel für frühgeborene (oder untergewichtige) Säuglinge, welche umfaßt:
(a) 8 bis 30 %, bezogen auf die Masse der Fettzusammensetzung, eines oder mehrerer Laurinsäureöle, ausgewählt aus Kokosnußöl, Babassuöl und Palmkernöl;
(b) 8 bis 32 %, bezogen auf die Masse der Fettzusammensetzung, eines oder mehrerer Palmitinsäureöle, ausgewählt aus Oleoöl, Palmöl und Palmoleinöl;
(c) 8 bis 30 %, bezogen auf die Masse der Fettzusammensetzung, eines oder mehrerer Oleinsäureöle, ausgewählt aus Olivenöl, Safloroleinöl, Sonnenblumenoleinöl und Canolaöl;
(d) 10 bis 30 %, bezogen auf die Masse der Fettzusammensetzung, eines oder mehrerer Linolsäureöle, ausgewählt aus Maisöl, Baumwollsamenöl, Safloröl, Sojabohnenöl und Sonnenblumenöl; und
(e) 10 bis 50 %, bezogen auf die Masse der FettzusammenSetzung, Triglyceride mittlerer Kettenlänge (MCTs),
worin zumindest das Palmitinsäureöl und das Laurinsäureöl co-randomisiert sind,
wobei die Mengen der Öle derart sind, daß die Fettzusammensetzung, pro 100 Masseteile der gesamten, als Triglyceride vorliegenden Fettsäuren, enthält:

(i) 8 bis 34 Teile Caprylsäure;

(ii) 4 bis 16 Teile Caprinsäure;

(iii) 5 bis 22 Teile Palmitinsäure;

(iv) 18 bis 37 Teile Oleinsäure; und

(v) 7 bis 19 Teile Linolsäure.

**11.** Co-randomisierte Fettzusammensetzung für Frühgeborene nach Anspruch 10, worin nur die Palmitinsäureöle und die Laurinsäureöle co-randomisiert sind.

**12.** Co-randomisierte Fettzusammensetzung für Frühgeborene nach Anspruch 10, worin nur ein Palmitinsäureöl und ein Laurinsäureöl co-randomisiert sind.

**13.** Co-randomisierte Fettzusammensetzung für Frühgeborene nach Anspruch 12, worin das Palmitinsäureöl Oleoöl oder Palmoleinöl ist, und das Laurinsäureöl Kokosnußöl ist.

**14.** Co-randomisierte Fettzusammensetzung für Frühgeborene nach Anspruch 10, worin das Palmitinsäureöl Palmoleinöl ist.

**15.** Co-randomisierte Fettzusammensetzung für Frühgeborene nach Anspruch 10, worin nur einer von jedem Öl-Typ verwendet wird.

**16.** Co-randomisierte Fettzusammensetzung für Frühgeborene nach Anspruch 10, worin ein Oleinsäureöl, das entweder Sonnenblumenoleinöl oder Safloroleinöl ist, verwendet wird.

**17.** Co-randomisierte Fettzusammensetzung für Frühgeborene nach Anspruch 10, worin nur ein Linolsäureöl, das Sojabohnenöl ist, verwendet wird.

**18.** Co-randomisierte Fettzusammensetzung, insbesondere zur Verwendung in einer Vollnahrungsmittelformel für frühgeborene (oder untergewichtige) Säuglinge, welche umfaßt:

(a) 15 bis 29 %, bezogen auf die Masse der Fettzusammensetzung, eines Laurinsäureöls, ausgewählt aus Kokosnußöl, Babassuöl und Palmkernöl;

(b) 15 bis 32 %, bezogen auf die Masse der Fettzusammensetzung, eines Palmitinsäureöls, ausgewählt aus Palmöl und Palmoleinöl;

(c) 8 bis 30 %, bezogen auf die Masse der Fettzusammensetzung, eines Oleinsäureöls, ausgewählt aus Olivenöl, Safloroleinöl, Sonnenblumenoleinöl und Canolaöl;

(d) 15 bis 27 %, bezogen auf die Masse der Fettzusammensetzung, eines Linolsäureöls, ausgewählt aus Maisöl, Baumwollsamenöl, Safloröl, Sojabohnenöl und Sonnenblumenöl; und

(e) 10 bis 30 %, bezogen auf die Masse der Fettzusammensetzung, Triglyceride mittlerer Kettenlänge (MCTs),

worin zumindest das Palmitinsäureöl und das Laurinsäureöl co-randomisiert sind,

wobei die Mengen der Öle derart sind, daß die Fettzusammensetzung, pro 100 Masseteile der gesamten, als Triglyceride vorliegenden Fettsäuren, enthält:

(i) 8 bis 25 Teile Caprylsäure;

(ii) 4 bis 12 Teile Caprinsäure;

(iii) 7 bis 20 Teile Palmitinsäure;

(iv) 25 bis 38 Teile Oleinsäure; und

(v) 12 bis 20 Teile Linolsäure.

**19.** Co-randomisierte Fettzusammensetzung nach Anspruch 1, worin die Öle umfassen:

(a) 22 bis 28 % Kokosnußöl;

(b) 30 bis 36 % Palmoleinöl;

(c) 21 bis 30 % Safloroleinöl oder Sonnenblumenoleinöl; und

(d) 14 bis 22 % Sojabohnenöl,

und worin die Fettzusammensetzung, pro 100 Masseteile der gesamten, als Triglyceride vorliegenden Fettsäuren, enthält:

(i) 8 bis 14 Teile Laurinsäure;

(ii) 15 bis 21 Teile Palmitinsäure;

(iii) 3 bis 5 Teile Stearinsäure;

(iv) 33 bis 43 Teile Oleinsäure; und

(v) 14 bis 21 Teile Linolsäure.

20. Co-randomisierte Fettzusammensetzung für Frühgeborene nach Anspruch 10, worin die Öle umfassen:

(a) 22 bis 28 % Kokosnußöl;

(b) 20 bis 30 % Palmoleinöl;

(c) 19 bis 30 % Safloroleinöl oder Sonnenblumenoleinöl; und

(d) 14 bis 22 % Sojabohnenöl; und

(e) 10 bis 30 % MCTs,

worin das Kokosnußöl und das Palmoleinöl co-randomisiert sind,

und worin die Fettzusammensetzung, pro 100 Masseteile der gesamten, als Triglyceride vorliegenden Fettsäuren, enthält:

(i) 8 bis 20 Teile Caprylsäure;

(ii) 4 bis 8 Teile Caprinsäure;

(iii) 10 bis 17 Teile Palmitinsäure;

(iv) 26 bis 36 Teile Oleinsäure; und

(v) 12 bis 20 Teile Linolsäure.

21. Vollnahrungsmittel-Zusammensetzung, adaptiert für die Ernährung von Säuglingen, welche eine Fettzusammensetzung nach einem der Ansprüche 1 bis 9 und 19, eine Protein-Quelle, eine Kohlenhydrat-Quelle, Vitamine und Mineralien umfaßt.

22. Vollnahrungsmittel-Zusammensetzung, adaptiert für die Ernährung frühgeborener Säuglinge oder untergewichtiger Säuglinge, welche eine Fettzusammensetzung nach einem der Ansprüche 10 bis 18 und 20, eine Protein-Quelle, eine Kohlenhydrat-Quelle, Vitamine und Mineralien umfaßt.

## Revendications

1. Composition de graisses transestérifiées, en particulier à utiliser dans une formulation nutritionnellement complète pour nouveau-nés, comprenant :

(a) 18 à 30%, calculés sur le poids de la composition de graisses, d'une ou de plusieurs huiles d'acide laurique, choisies parmi l'huile de noix de coco, l'huile de babassu et l'huile de noyaux de palme;

(b) 20 à 40%, calculés sur le poids de la composition de graisses, d'une ou de deux huiles d'acide palmitique, choisies parmi l'huile oléo, l'huile de palme et l'huile oléine de palme;

(c) 13 à 34%, calculés sur le poids de la composition de graisses, d'une ou de plusieurs huiles d'acide oléique, choisies parmi l'huile d'olive, l'huile oléique de carthame, l'huile oléique de tournesol et l'huile de canola, et

(d) 12 à 27%, calculés sur le poids de la composition de graisses, d'une ou de plusieurs huiles d'acide linoléique, choisies parmi l'huile de maïs, l'huile de coton, l'huile de carthame, l'huile de soja et l'huile de tournesol,

où au moins les huiles d'acide palmitique et les huiles d'acide laurique sont transestérifiées, les quantités des huiles étant telles que la composition de graisse contient, par 100 parties en poids des acides gras totaux présents sous forme de triglycérides :

(i) 9 à 20 parties d'acide laurique;

(ii) 10 à 25 parties d'acide palmitique;

(iii) 2 à 10 parties d'acide stéarique;

(iv) 25 à 45 parties d'acide oléique, et

(v) 11 à 28 parties d'acide linoléique.

2. Composition de graisses transestérifiées suivant la revendication 1, dans laquelle seules les huiles d'acide palmitique et les huiles d'acide laurique sont transestérifiées.

3. Composition de graisses transestérifiées suivant la revendication 2, dans laquelle seules une huile d'acide palmitique et une huile d'acide laurique sont transestérifiées.

4. Composition de graisses transestérifiées suivant la revendication 3, dans laquelle l'huile d'acide palmitique est l'huile oléo ou l'huile oléine de palme et l'huile d'acide laurique est l'huile de noix de coco.

5. Composition de graisses transestérifiées suivant la revendication 1, dans laquelle un type seulement de chaque huile est utilisé.

6. Composition de graisses transestérifiées suivant la revendication 1, dans laquelle une huile d'acide palmitique est utilisée, qui est l'huile oléine de palme.

7. Composition de graisses transestérifiées suivant la revendication 1, dans laquelle une huile d'acide oléique est utilisée, qui est soit l'huile oléique de tournesol, soit l'huile oléique de carthame.

8. Composition de graisses transestérifiées suivant la revendication 1, dans laquelle une huile d'acide linoléique seulement est utilisée, qui est l'huile de soja.

9. Composition de graisses transestérifiées, en particulier à utiliser dans une formulation nutritionnellement complète pour nouveau-nés, comprenant :
   (a) 20 à 29%, calculés sur le poids de la composition de graisses, d'une huile d'acide laurique, choisie parmi l'huile de noix de coco, l'huile de babassu et l'huile de noyaux de palme;
   (b) 26 à 38%, calculés sur le poids de la composition de graisses, d'une huile d'acide palmitique, choisie parmi l'huile de palme et l'huile oléine de palme;
   (c) 14 à 30%, calculés sur le poids de la composition de graisses, d'une huile d'acide oléique, choisie parmi l'huile d'olive, l'huile oléique de carthame, l'huile oléique de tournesol et l'huile de canola;
   (d) 14 à 27%, calculés sur le poids de la composition de graisses, d'une huile d'acide linoléique, choisie parmi l'huile de maïs, l'huile de coton, l'huile de carthame, l'huile de soja et l'huile de tournesol,
   où au moins l'huile d'acide palmitique et l'huile d'acide laurique sont transestérifiées, les quantités des huiles étant telles que la composition de graisses contient, par 100 parties en poids des acides gras totaux présents sous forme de triglycérides :
   (i) 10 à 17 parties d'acide laurique;
   (ii) 11 à 22 parties d'acide palmitique
   (iii) 3 à 8 parties d'acide stéarique;
   (iv) 30 à 43 parties d'acide oléique, et
   (v) 13 à 23 parties d'acide linoléique.

10. Composition de graisses transestérifiées, en particulier à utiliser dans une formulation nutritionnellement complète pour nouveau-nés prématurés (ou de poids de naissance faible), comprenant :
   (a) 8 à 30%, calculés sur le poids de la composition de graisses, d'une ou de plusieurs huiles d'acide laurique, choisies parmi l'huile de coco, l'huile de babassu et l'huile de noyaux de palme;
   (b) 8 à 32%, calculés sur le poids de la composition de graisses, d'une ou de plusieurs huiles d'acide palmitique, choisies parmi l'huile oléo, l'huile de palme et l'huile oléine de palme;
   (c) 8 à 30%, calculés sur le poids de la composition de graisses, d'une ou de plusieurs huiles d'acide oléique, choisies parmi l'huile d'olive, l'huile oléique de carthame, l'huile oléique de tournesol et l'huile de canola;
   (d) 10 à 30%, calculés sur le poids de la composition de graisses, d'une ou de plusieurs huiles d'acide linoléique, choisies parmi l'huile de maïs, l'huile de coton, l'huile de carthame, l'huile de soja et l'huile de tournesol, et
   (e) 10 à 50%, calculés sur le poids de la composition de graisses, de triglycérides à chaîne moyenne (TCM);
   où au moins l'huile d'acide palmitique et l'huile d'acide laurique sont transestérifiées, les quantités des huiles étant telles que la composition de graisses contient, par 100 parties en poids des acides gras totaux présents sous forme de triglycérides :
   (i) 8 à 34 parties d'acide caprylique;
   (ii) 4 à 16 parties d'acide caprique;
   (iii) 5 à 22 parties d'acide palmitique;
   (iv) 18 à 37 parties d'acide oléique, et
   (v) 7 à 19 parties d'acide linoléique.

22

**11.** Composition de graisses transestérifiées, pour prématurés, suivant la revendication 10, dans laquelle seules les huiles d'acide palmitique et les huiles d'acide laurique sont transestérifiées.

**12.** Composition de graisses transestérifiées, pour prématurés, suivant la revendication 10, dans laquelle seules une huile d'acide palmitique et une huile d'acide laurique sont tranestérifiées.

**13.** Composition de graisses transestérifiées, pour prématurés, suivant la revendication 12, dans laquelle l'huile d'acide palmitique est l'huile oléo ou l'huile oléine de palme et l'huile d'acide laurique est l'huile de noix de coco.

**14.** Composition de graisses transestérifiées, pour prématurés, suivant la revendication 10, dans laquelle l'huile d'acide palmitique est l'huile oléine de palme.

**15.** Composition de graisses transestérifiées, pour prématurés, suivant la revendication 10, dans laquelle un type seulement de chaque huile est utilisé.

**16.** Composition de graisses transestérifiées, pour prématurés, suivant la revendication 10, dans laquelle une huile d'acide oléique est utilisée, qui est soit l'huile oléique de tournesol, soit l'huile oléique de carthame.

**17.** Composition de graisses transestérifiées, pour prématurés, suivant là revendication 10, dans laquelle une huile d'acide linoléique seulement est utilisée, qui est l'huile de soja.

**18.** Composition de graisses transestérifiées, en particulier à utiliser dans une formulation nutritionnellement complète pour nouveau-nés prématurés (ou de poids de naissance faible), comprenant :
   (a) 15 à 29%, calculés sur le poids de la composition de graisses, d'une huile d'acide laurique, choisie parmi l'huile de noix de coco, l'huile de babassu et l'huile de noyaux de palme;
   (b) 15 à 32%, calculés sur le poids de la composition de graisses, d'une huile d'acide palmitique, choisie parmi l'huile de palme et l'huile oléine de palme;
   (c) 8 à 30%, calculés sur le poids de la composition de graisses, d'une huile d'acide oléique, choisie parmi l'huile d'olive, l'huile oléique de carthame, l'huile oléique de tournesol et l'huile de canola;
   (d) 15 à 27%, calculés sur le poids de la composition de graisses, d'une huile d'acide linoléique, choisie parmi l'huile de maïs, l'huile de coton, l'huile de carthame, l'huile de soja et l'huile de tournesol, et
   (e) 10 à 30%, calculés sur le poids de la composition de graisses, de triglycérides à chaîne moyenne (TCM);
   où au moins l'huile d'acide palmitique et l'huile d'acide laurique sont transestérifiées, les quantités des huiles étant telles que la composition de graisses contient, par 100 parties en poids, des acides gras totaux présents sous forme de triglycérides :
   (i) 8 à 25 parties d'acide caprylique;
   (ii) 4 à 12 parties d'acide caprique;
   (iii) 7 à 20 parties d'acide palmitique;
   (iv) 25 à 38 parties d'acide oléique, et
   (v) 12 à 20 parties d'acide linoléique.

**19.** Composition de graisses transestérifiées suivant la revendication 1, dans laquelle les huiles comprennent :
   (a) 22 à 28% d'huile de noix de coco;
   (b) 30 à 36% d'huile oléine de palme;
   (c) 21 à 30% d'huile oléique de carthame ou d'huile oléique de tournesol, et
   (d) 14 à 22% d'huile de soja, et
   dans laquelle le composition de graisses contient, par 100 parties en poids, des acides gras totaux présents sous forme de triglycérides :
   (i) 8 à 14 parties d'acide laurique;
   (ii) 15 à 21 parties d'acide palmitique;
   (iii) 3 à 5 parties d'acide stéarique;
   (iv) 33 à 43 parties d'acide oléique, et
   (v) 14 à 21 parties d'acide linoléique.

**20.** Composition de graisses transestérifiées, pour prématurés, suivant la revendication 10, dans laquelle les huiles comprennent :

(a) 22 à 28% d'huile de noix de coco;

(b) 20 à 30% d'huile oléine de palme;

(c) 19 à 30% d'huile oléique de carthame ou d'huile oléique de tournesol;

(d) 14 à 22% d'huile de soja, et

(e) 10 à 30% de TCM,

dans laquelle l'huile de noix de coco et l'huile oléine de palme sont transestérifiées et dans laquelle le composition de graisses contient, par 100 parties en poids, des acides gras totaux présents sous forme de triglycérides :

(i) 8 à 20 parties d'acide caprylique;

(ii) 4 à 8 parties d'acide caprique;

(iii) 10 à 17 parties d'acide palmitique;

(iv) 26 à 36 parties d'acide oléique, et

(v) 12 à 20 parties d'acide linoléique.

**21.** Composition alimentaire nutritionnellement complète adaptée à l'alimentation de nouveau-nés humains, qui comprend une composition de graisses suivant l'une quelconque des revendications 1 à 9 et 19, une source de protéine, une source d'hydrate de carbone, des vitamines et des minéraux.

**22.** Composition alimentaire nutritionnellement complète, adaptée à l'alimentaiton de nouveau-nés prématurés ou de nouveau-nés de poids de naissance faible, qui comprend une composition de graisses suivant l'une quelconque des revendications 10 à 18 et 20, une source de protéine, une source d'hydrate de carbone, des vitamines et des minéraux.